# EUROPEAN PATENT SPECIFICATION

(11) **EP 3 677 254 B1**
(45) Date of publication and mention of the grant of the patent: **25.10.2023**
(21) Application number: 19219722.6
(22) Date of filing: 26.12.2019
(51) Int. Cl.: A61K 9/20, A61K 9/28, A61K 31/137

(54) **SOLID ORAL PHARMACEUTICAL COMPOSITIONS OF DESVENLAFAXINE**
FESTE ORALE PHARMAZEUTISCHE ZUSAMMENSETZUNGEN VON DESVENLAFAXIN
COMPOSITIONS PHARMACEUTIQUES ORALES SOLIDES DE DESVENLAFAXINE

(30) Priority: 27.12.2018 TR 201820634
(43) Date of publication of application: 08.07.2020
(73) Proprietor: Sanovel Ilac Sanayi ve Ticaret A.S., 34460 Sariyer/Istanbul (TR)
(72) Inventor: TÜRKOGLU, Damla, 34460 Istanbul (TR); TOMBAYOGLU, Vildan, 34460 Istanbul (TR); PALANTÖKEN, Arzu, 34460 Istanbul (TR); TÜRKYILMAZ, Ali, 34460 Istanbul (TR)
(74) Representative: Sevinç, Erkan

(56) References cited:
- WO-A1-2012/140577
- US-A1- 2012 087 986

## Description

### Field of Invention

The present invention relates to solid oral pharmaceutical compositions comprising desvenlafaxine succinate monohydrate which provides enhanced solubility and stability.

### Background of Invention

Desvenlafaxine is a cyclohexanol and phenol derivative and metabolite of venlafaxine that functions as a serotonin and noradrenaline reuptake inhibitor (SNRI) and is used as an antidepressive agent. By blocking both transporters, this agent prolongs neurotransmitter activities of both serotonin and norepinephrine, thereby alleviating depressive state. It is being targeted as the first non-hormonal based treatment for menopause.

Desvenlafaxine's primary use in medicine is the treatment of major depressive disorder. In clinical studies, doses of 50-400 mg/day were shown to be effective for the major depressive disorder (MDD), although no additional benefit was demonstrated at doses greater than 50 mg/day, and adverse events and discontinuations were more frequent at higher doses.

Desvenlafaxine, also called O-desmethyl-venlafaxine, is a synthetic form of the isolated major active metabolite of venlafaxine. When most normal metabolizers take venlafaxine, approximately 70% of the dose is metabolized into desvenlafaxine, so the effects of the two drugs are expected to be very similar. It works by blocking the "reuptake" transporters for key neurotransmitters affecting mood, thereby leaving more active neurotransmitters in the synapse. The neurotransmitters affected are serotonin (5-hydroxytryptamine) and norepinephrine (noradrenaline). It is approximately 10 times more potent at inhibiting serotonin uptake than norepinephrine uptake. It is marketed under the brand name Pristiq^{®} by Wyeth (now part of Pfizer).

Its chemical name is 4-[2-(dimethylamino)-1-(1-hydroxycyclohexyl)ethyl]phenol and its chemical structure is shown in Formula I.

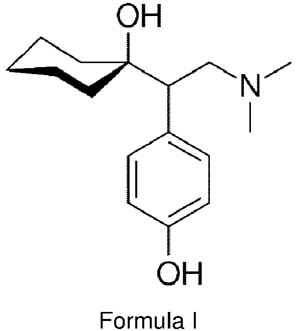

Desvenlafaxine is generally used as its salts in the formulations to provide an effective dissolution. Salt formation provides a means of altering the physicochemical and resultant biological characteristics of a drug without modifying its chemical structure. A salt form can have a dramatic influence on the properties of the drug. Hygroscopicity, stability and solubility are some of the physical parameters which can be enhanced by the selection of a suitable salt.

Different salts of desvenlafaxine have been disclosed in the literature. Succinate, formate, hydrochloride, citrate, maleate, mesylate, mandelate, quinic acid salt and tartrate, phosphate, glucuronate, orotate, oxalate, benzoate, lactate, saccharinate salts are some of them.

In the prior art, patent document numbered EP2367536B1 discloses sustained release pharmaceutical compositions comprising O-desmethyl-venlafaxine, in particular, sustained pharmaceutical compositions comprising O-desmethyl-venlafaxine orotate and / or O-desmethyl-venlafaxine glucuronate and a release-rate controlling polymer. The object of the invention subjected to this patent document is to provide pharmaceutical compositions suitable and beneficial for sustained release of O-desmethyl-venlafaxine active agent by adjusting the amount of the release-rate controlling polymer in the composition.

Patent application numbered WO03103603A1A discloses formate salt of desvenlafaxine for the first time and also presents pharmaceutical compositions comprising desvenlafaxine formate in crystalline form.

US2007037884A1, another patent document in the art, mentions tablet and capsule formulations comprising succinate salt of desvenlafaxine. The document promotes the use of hydroxypropyl methylcellulose (hypromellose) as rate controlling polymer in high amounts, more specifically in an amount of not less than 50% by weight of the total composition. Hypromellose is a highly hygroscopic material and also it is known that excessive oral consumption may have a laxative effect (Handbook of Pharmaceutical Excipients edited by Raymond C. Rowe, Paul J. Sheskey and Marian E. Quinn, sixth edition, page 328).

In the patent literature, desvenlafaxine succinate is also present in dosage forms other than tablets and capsules, such as hydrogels and solid dispersions. For example, in the patent application numbered US2010210719A1, stable amorphous O-desmethylvenlafaxine succinate solid dispersions with one or more pharmaceutically acceptable carriers are suggested.

US 2012/087986 A1 relates to formulations comprising desvenlafaxine, and processes for preparing the formulations. Desvenlafaxine succinate formulations are mentioned in US 2012/087986 A1. Dissolution profile of the active agent is one of the aspects referred in US 2012/087986 A1. Desired modified release profile is obtained by the use of release rate controlling polymers. According to US 2012/087986 A1; the formulation may comprise a premix of desvenlafaxine succinate and povidone, Kollidon SR, Eudragit, dibasic calcium phosphate dihydrate, castor oil, Carbopol, microcrystalline cellulose, talc and magnesium stearate.

In the light of the prior art, there is still a need for tablet and capsule formulations of desvenlafaxine succinate eliminating laxative effect and providing enhanced stability and reduced hygroscopicity at the same time by the selection of suitable salts and excipients.

### Objects and Brief Description of the Invention

The main object of the present invention is to obtain solid oral pharmaceutical compositions of desvenlafaxine eliminating all aforesaid problems and bringing additional advantages to the relevant prior art.

Another object of the present invention is to develop solid oral pharmaceutical compositions of desvenlafaxine with reduced hygroscopicity.

Another object of the present invention is to develop solid oral pharmaceutical compositions of desvenlafaxine with enhanced stability.

Another object of the present invention is to obtain solid oral pharmaceutical compositions of desvenlafaxine with increased uniformity.

Another object of the present invention is to obtain solid oral pharmaceutical compositions of desvenlafaxine with enhanced dissolution profile and bioavailability.

A further object of the present invention is to develop tablet and capsule compositions comprising desvenlafaxine succinate.

A further object of the present invention is to develop tablet and capsule compositions of desvenlafaxine which is free of carrier and hypromellose.

Yet another object of the present invention is to improve a process for preparing the said tablet and capsule compositions, comprising wet granulation method which makes round and compact granules having a high content uniformity and a narrow range of particle sizes.

Yet another object of the present invention is to improve a process for preparing the said tablet and capsule compositions, comprising spray drying method which provides effective encapsulation and homogeneity for the granules.

### Description of the Invention

In accordance with the objects outlined above, detailed features of the present invention are given herein.

The present invention relates to a solid oral pharmaceutical composition comprising desvenlafaxine succinate monohydrate.

In an embodiment, said desvenlafaxine succinate is in amorphous or crystalline or polymorphous form. According to the most preferred embodiment, the composition comprises desvenlafaxine succinate monohydrate in amorphous form.

The composition is in the form of coated tablet, trilayer tablet, bilayer tablet, multilayer tablet, orally disintegrating tablet, mini tablet, pellet, sugar pellet, buccal tablet, sublingual tablet, effervescent tablet, immediate release tablet, modified release tablet, film-coated tablet, gastric disintegrating tablet, pill, capsule, oral granule, powder, coated bead system, microsphere, tablet in tablet, inlay tablet, dragee, sachet or orally administrable film.

According to the preferred embodiment, the composition is in the film-coated tablet dosage form.

According to another preferred embodiment, the composition is in the capsule dosage form.

The amount of desvenlafaxine succinate monohydrate in an amount of 5-70%, preferably 40-60%, more preferably 50-55% by weight of the composition.

According to the preferred embodiment, the composition is free of hydroxypropyl methylcellulose (hypromellose) which is known as a highly hygroscopic material, also as causing laxative effect. The composition is also free of starch which is highly hygroscopic. Accordingly, patient compatibility and stability of the composition is enhanced.

According to one embodiment, the composition further comprises at least one pharmaceutically acceptable excipient selected from release-rate controlling agents, diluents, binders, lubricants, glidants or mixtures thereof.

According to the preferred embodiment, the composition is also free of disintegrant.

According to one embodiment, the solid oral pharmaceutical composition comprises at least one release-rate controlling agent which is selected from the group comprising hydroxy alkyl cellulose, cellulose acetate, lauroyl polyoxyl glycerides, polyethylene oxide, alkyl cellulose, carboxymethyl cellulose, polyethylene glycol, polysaccharides, sodium alginate, propylene glycol alginate, xanthan gum, carrageenans, acrylic polymers and copolymers, carbomers, polymethacrylates, polyvinyl acetate/povidone (Kollidon SR), glyceryl behenate, ethylene vinyl acetate copolymer or mixtures thereof.

The amount of release-rate controlling agent is between 10-60%, preferably 20-50% by weight of the total composition.

According to one preferred embodiment, the solid oral pharmaceutical composition comprises two release-rate controlling agents which are glyceryl behenate and polyvinyl acetate and povidone-based matrix agent. Said matrix agent comprises 70-90% polyvinyl acetate and 10-30% povidone by weight of the matrix agent.

According to another preferred embodiment, the solid oral pharmaceutical composition comprises a release-rate controlling agent which is carbomer.

According to one embodiment, the solid oral pharmaceutical composition comprises at least one binder which is selected from the group comprising lactose monohydrate, copovidone, povidone, carnauba wax, pullulan, polymethacrylate, glyceryl behenate, hydroxypropyl cellulose (HPC), carboxymethyl cellulose (CMC), methyl cellulose (MC), hydroxyethyl cellulose, sodium carboxymethyl cellulose (Na CMC), carboxymethyl cellulose calcium, ethyl cellulose, polymetacrylates, polyethylene oxide, polyvinyl alcohol, polycarbophil, polyvinyl acetate and its copolymers, xanthan gum, guar gum, alginate, carrageen, collagen, agar, pectin, hyaluronic acid, carbomer, cellulose acetate phthalate, poloxamer, polyethylene glycol (PEG), sugars, glycose syrups, natural gums, tragacanth gum, polyacrylamide, aluminum hydroxide, benthonite, laponite, setostearyl alcohol, polyoxyethylene-alkyl ethers, acacia mucilage, polydextrose or mixtures thereof.

In the preferred embodiment of the invention, the ratio of binder to the release-rate controlling agent is in the range of 1:0.2 to 1:60 and preferably 1:0.5 to 1:10. This preferred selection of range assures the enhanced bioavailability and stability during the shelf life.

According to one preferred embodiment, the solid oral pharmaceutical composition comprises one binder which is lactose monohydrate. The amount of lactose monohydrate is between 1-50%, preferably 3-40% by weight of the total composition. This amount varies depending on the effectiveness and ratio of the release-rate controlling agent.

According to one embodiment, the solid oral pharmaceutical composition comprises at least one lubricant and at least one glidant which are selected from the group comprising sodium lauryl sulphate, sodium stearyl fumarate, colloidal silicon dioxide, zinc stearate, calcium stearate, mineral oil, talc, polyethylene glycol, glyceryl monostearate, glyceryl palmitostearate, magnesium lauryl sulphate, fumaric acid, zinc stearate, stearic acid, hydrogenated natural oils, silica, paraffin or mixtures thereof.

According to the preferred embodiment, the composition is free of magnesium stearate because of its high hygroscopicity and its negative effects on the stability of the solid dosage form.

The amount of lubricant is between 0.1-10%, preferably 1-5% by weight of the total composition.

According to the preferred embodiment, the solid oral pharmaceutical composition comprises one lubricant which is sodium stearyl fumarate.

The amount of glidant is between 0.1-10%, preferably 0.5-3% by weight of the total composition.

According to the preferred embodiment, the solid oral pharmaceutical composition comprises one glidant which is colloidal silicon dioxide.

According to one embodiment, the solid oral pharmaceutical composition comprises at least one coating to protect the composition against the moisture and maintain the stability. Suitable coating ingredients are selected from the group comprising hydroxypropylmethyl cellulose (hypromellose), hydroxypropyl cellulose, polyvinyl alcohol (PVA), polyethylene glycol (PEG), talc, polyvinyl alcohol-polyethylene glycol copolymers (Kollicoat IR), ethylcellulose dispersions (Surelease), polyvinylpyrrolidone, polyvinylpyrrolidone-vinyl acetate copolymer (PVP-VA) and all kinds of Opadry^{®}, pigments, dyes, titanium dioxide, iron oxide, polymethylmetacrylate copolymers (Eudragit) or mixtures thereof.

According to the preferred embodiment, the coating comprises polyvinyl alcohol, polyethylene glycol, titanium dioxide, talc, iron oxide.

According to this preferred embodiment, the coating comprises;
- 20-60% by weight of polyvinyl alcohol,
- 10-30% by weight of polyethylene glycol,
- 10-30% by weight of titanium dioxide,
- 10-30% by weight of talc,
- 0.1-5% iron oxide.

The amount of coating is between 1-10%, preferably 1-5% by weight of the total composition.

According to one embodiment, the solid oral pharmaceutical composition comprises;
- 5-70% by weight of desvenlafaxine succinate monohydrate,
- 5-30% by weight of glyceryl behenate,
- 5-30% by weight of a matrix agent which comprises polyvinyl acetate in an amount of 70-90% and povidone in an amount of 10-30%,
- 1-50% by weight of lactose monohydrate,
- 0.1-10% by weight of sodium stearyl fumarate,
- 0.1-10% by weight of colloidal silicon dioxide.

These analytically selected excipients and ratios ensure the required effective doses for the treatment and enhance stability and dissolution profile of the film-coated tablet subjected to the invention.

According to this embodiment, the below given formulations can be used in the solid oral pharmaceutical composition subjected to the invention.

### Example 1: Capsule formulation

| **Ingredients** | **Amount (%)** |
|---|---|
| Desvenlafaxine succinate monohydrate | 50.0 - 55.0 |
| Glyceryl behenate | 10.0 - 20.0 |
| PVA and povidone based matrix agent | 20.0 - 30.0 |
| Lactose monohydrate | 3.0 - 10.0 |
| Sodium stearyl fumarate | 1.0 - 5.0 |
| Colloidal silicon dioxide | 0.5 - 3.0 |
| **Total** | **100.0** |

### Example 2: Capsule formulation

| **Ingredients** | **Amount (%)** |
|---|---|
| Desvenlafaxine succinate monohydrate | 52.4 |
| Glyceryl behenate | 15.0 |
| PVA and povidone based matrix agent | 25.0 |
| Lactose monohydrate | 4.6 |
| Sodium stearyl fumarate | 1.0 |
| Colloidal silicon dioxide | 2.0 |
| **Total** | **100.0** |

The pharmaceutical formulations mentioned above in Example 1 and Example 2 are prepared by following these steps:
- Sieving and mixing desvenlafaxine succinate monohydrate, glyceryl behenate, PVA and povidone based matrix agent, lactose monohydrate and colloidal silicon dioxide
- Adding sodium stearyl fumarate and mixing
- Filling the final powder mixture into capsules

### Example 3: Film-coated tablet formulation

| **Ingredients** | **Amount (%)** |
|---|---|
| Desvenlafaxine succinate monohydrate | 50.0 - 55.0 |
| Glyceryl behenate | 10.0 - 20.0 |
| PVA and povidone based matrix agent | 20.0 - 30.0 |
| Lactose monohydrate | 3.0 - 10.0 |
| Sodium stearyl fumarate | 1.0 - 5.0 |
| Colloidal silicon dioxide | 0.5 - 3.0 |
| **Total** | **100.0** |
| Film coating | 1.0 - 5.0 |

| **Coating** | **Amount** (**%)** |
|---|---|
| Polyvinyl alcohol | 20.0-60.0 |
| Polyethylene glycol | 10.0-30.0 |
| Titanium dioxide | 10.0-30.0 |
| Talc | 10.0-30.0 |
| Iron oxide | 0.1-5.0 |
| **Total Coating** | **100.0** |

### Example 4: Film-coated tablet formulation

| **Ingredients** | **Amount (%)** |
|---|---|
| Desvenlafaxine succinate monohydrate | 52.4 |
| Glyceryl behenate | 15.0 |
| PVA and povidone based matrix agent | 25.0 |
| Lactose monohydrate | 4.6 |
| Sodium stearyl fumarate | 1.0 |
| Colloidal silicon dioxide | 2.0 |
| **Total** | **100.0** |
| Film coating | 3.0 |

The pharmaceutical formulations mentioned above in Example 3 and Example 4 are prepared by following these steps:
- Sieving and mixing desvenlafaxine succinate monohydrate, glyceryl behenate, PVA and povidone based matrix agent, lactose monohydrate and colloidal silicon dioxide
- Adding sodium stearyl fumarate and mixing
- Compressing the final mixture into tablets

According to one embodiment, the solid oral pharmaceutical composition comprises;
- 5-70% by weight of desvenlafaxine succinate monohydrate,
- 10-60% by weight of carbomer
- 1-50% by weight of lactose monohydrate,
- 0.1-10% by weight of sodium stearyl fumarate,
- 0.1-10% by weight of colloidal silicon dioxide.

These analytically selected excipients and ratios ensure the required effective doses for the treatment and enhance stability and dissolution profile of the film-coated tablet subjected to the invention.

According to this embodiment, the below given formulations can be used in the solid oral pharmaceutical composition subjected to the invention.

### Example 5: Capsule formulation

| **Ingredients** | **Amount (%)** |
|---|---|
| Desvenlafaxine succinate monohydrate | 50.0 - 55.0 |
| Carbomer | 10.0 - 20.0 |
| Lactose monohydrate | 20.0 - 45.0 |
| Sodium stearyl fumarate | 1.0 - 5.0 |
| Colloidal silicon dioxide | 0.5 - 3.0 |
| **Total** | **100.0** |

### Example 6: Capsule formulation

| **Ingredients** | **Amount (%)** |
|---|---|
| Desvenlafaxine succinate monohydrate | 52.4 |
| Carbomer | 15.0 |
| Lactose monohydrate | 29.6 |
| Sodium stearyl fumarate | 1.0 |
| Colloidal silicon dioxide | 2.0 |
| **Total** | **100.0** |

The pharmaceutical formulations mentioned above in Example 5 and Example 6 are prepared by following these steps:
- Sieving and mixing desvenlafaxine succinate monohydrate, carbomer and lactose monohydrate with the addition of water
- Drying the mixture in an incubator and cooling
- Adding colloidal silicon dioxide into the mixture and mixing
- Adding sodium stearyl fumarate into the mixture and mixing
- Filling the final powder mixture into capsules

### Example 7: Film-coated tablet formulation

| **Ingredients** | **Amount (%)** |
|---|---|
| Desvenlafaxine succinate monohydrate | 50.0 - 55.0 |
| Carbomer | 10.0 - 20.0 |
| Lactose monohydrate | 20.0 - 45.0 |
| Sodium stearyl fumarate | 1.0 - 5.0 |
| Colloidal silicon dioxide | 0.5 - 3.0 |
| **Total** | **100.0** |
| Film coating | 1.0 - 5.0 |

| **Coating** | **Amount (%)** |
|---|---|
| Polyvinyl alcohol | 20.0-60.0 |
| Polyethylene glycol | 10.0-30.0 |
| Titanium dioxide | 10.0-30.0 |
| Talc | 10.0-30.0 |
| Iron oxide | 0.1-5.0 |
| **Total Coating** | **100.0** |

### Example 8: Film-coated tablet formulation

| **Ingredients** | **Amount (%)** |
|---|---|
| Desvenlafaxine succinate monohydrate | 52.4 |
| Carbomer | 15.0 |
| Lactose monohydrate | 29.6 |
| Sodium stearyl fumarate | 1.0 |
| Colloidal silicon dioxide | 2.0 |
| **Total** | **100.0** |
| Film coating | 3.0 |

The pharmaceutical formulations mentioned above in Example 7 and Example 8 are prepared by following these steps:
- Sieving and mixing desvenlafaxine succinate monohydrate, carbomer and lactose monohydrate with the addition of water
- Drying the mixture in an incubator and cooling
- Adding colloidal silicon dioxide into the mixture and mixing
- Adding sodium stearyl fumarate into the mixture and mixing
- Compressing the final mixture into tablets

Wet granulation method which is performed to granulate the powder mixture results in granules with a round, compact structure, excellent flow properties, a high bulk density and a narrow range of particle sizes. These properties make easier to process and enable the use of the selected excipients mentioned above. In addition to this, spray granulation with fluid bed technology enables to choose residual moisture and particle size. Consequently, the dosage form has an enhanced uniformity and stability with a prolonged shelf-life.

## Claims

1. A solid oral pharmaceutical formulation comprising desvenlafaxine succinate monohydrate.

2. The solid oral pharmaceutical formulation according to claim 1, wherein the composition is in the form of coated tablet, trilayer tablet, bilayer tablet, multilayer tablet, orally disintegrating tablet, mini tablet, pellet, sugar pellet, buccal tablet, sublingual tablet, effervescent tablet, immediate release tablet, modified release tablet, film-coated tablet, gastric disintegrating tablet, pill, capsule, oral granule, powder, coated bead system, microsphere, tablet in tablet, inlay tablet, dragee, sachet or orally administrable film.

3. The solid oral pharmaceutical formulation according to claim 2, wherein the composition is in the film-coated tablet dosage form.

4. The solid oral pharmaceutical formulation according to claim 2, wherein the composition is in the capsule dosage form.

5. The solid oral pharmaceutical formulation according to any preceding claim, wherein the composition further comprises at least one pharmaceutically acceptable excipient selected from release-rate controlling agents, diluents, binders, lubricants, glidants or mixtures thereof.

6. The solid oral pharmaceutical formulation according to claim 5, wherein the composition comprises at least one release-rate controlling agent which is selected from the group comprising hydroxy alkyl cellulose, polyethylene oxide, alkyl cellulose, carboxymethyl cellulose, polyethylene glycol, polysaccharides, sodium alginate, propylene glycol alginate, xanthan gum, carrageenans, acrylic polymers and copolymers, carbomers, polymethacrylates, polyvinyl acetate/povidone, glyceryl behenate, ethylene vinyl acetate copolymer or mixtures thereof.

7. The solid oral pharmaceutical formulation according to claim 6, wherein the amount of release-rate controlling agent is selected from the group comprising glyceryl behenate, polyvinyl acetate and povidone-based matrix and carbomer.

8. The solid oral pharmaceutical formulation according to claim 6 or 7, wherein the composition comprises at least one binder which is selected from the group comprising lactose monohydrate, copovidone, povidone, carnauba wax, pullulan, polymethacrylate, glyceryl behenate, hydroxypropyl cellulose (HPC), carboxymethyl cellulose (CMC), methyl cellulose (MC), hydroxyethyl cellulose, sodium carboxymethyl cellulose (Na CMC), carboxymethyl cellulose calcium, ethyl cellulose, polymetacrylates, polyethylene oxide, polyvinyl alcohol, polycarbophil, polyvinyl acetate and its copolymers, xanthan gum, guar gum, alginate, carrageen, collagen, agar, pectin, hyaluronic acid, carbomer, cellulose acetate phthalate, poloxamer, polyethylene glycol (PEG), sugars, glycose syrups, natural gums, tragacanth gum, polyacrylamide, aluminum hydroxide, benthonite, laponite, setostearyl alcohol, polyoxyethylene-alkyl ethers, acacia mucilage, polydextrose or mixtures thereof.

9. The solid oral pharmaceutical formulation according to claim 8, the ratio of the binder to the release-rate controlling agent is in the range of 1:0.2 to 1:60 and preferably 1:0.5 to 1:10.

10. The solid oral pharmaceutical composition according to claim 3, wherein the composition comprises;
- 5-70% by weight of desvenlafaxine succinate monohydrate,
- 5-30% by weight of glyceryl behenate,
- 5-30% by weight of a matrix agent which comprises polyvinyl acetate in an amount of 70-90% and povidone in an amount of 10-30%,
- 1-50% by weight of lactose monohydrate,
- 0.1-10% by weight of sodium stearyl fumarate,
- 0.1-10% by weight of colloidal silicon dioxide.

11. The solid oral pharmaceutical composition according to claim 3, wherein the composition comprises;
- 5-70% by weight of desvenlafaxine succinate monohydrate,
- 10-60% by weight of carbomer
- 1-50% by weight of lactose monohydrate,
- 0.1-10% by weight of sodium stearyl fumarate,
- 0.1-10% by weight of colloidal silicon dioxide.

12. The solid oral pharmaceutical formulation according to claim 10 or 11, the composition further comprises a coating comprising polyvinyl alcohol, polyethylene glycol, titanium dioxide, talc, iron oxide.

13. The solid oral pharmaceutical composition according to claim 4, wherein the composition comprises;
- 5-70% by weight of desvenlafaxine succinate monohydrate,
- 5-30% by weight of glyceryl behenate,
- 5-30% by weight of a matrix agent which comprises polyvinyl acetate in an amount of 70-90% and povidone in an amount of 10-30%,
- 1-50% by weight of lactose monohydrate,
- 0.1-10% by weight of sodium stearyl fumarate,
- 0.1-10% by weight of colloidal silicon dioxide.

14. The solid oral pharmaceutical composition according to claim 4, wherein the composition comprises;
- 5-70% by weight of desvenlafaxine succinate monohydrate,
- 10-60% by weight of carbomer
- 1-50% by weight of lactose monohydrate,
- 0.1-10% by weight of sodium stearyl fumarate,
- 0.1-10% by weight of colloidal silicon dioxide.

## Patentansprüche

1. Feste orale pharmazeutische Formulierung, aufweisend Desvenlafaxinsuccinat-Monohydrat.

2. Feste orale pharmazeutische Formulierung nach Anspruch 1, wobei die Zusammensetzung in der Form einer beschichteten Tablette, einer dreischichtigen Tablette, einer zweischichtigen Tablette, einer mehrschichtigen Tablette, einer sich oral zersetzenden Tablette, einer Minitablette, eines Pellets, eines Zuckerpellets, einer bukkalen Tablette, einer sublingualen Tablette, einer Brausetablette, einer Tablette mit sofortiger Freisetzung, einer Tablette mit modifizierter Freisetzung, einer Filmtablette, einer sich im Magen zersetzenden Tablette, einer Pille, einer Kapsel, eines oralen Granulats, eines Pulvers, eines beschichteten Kügelchensystems, einer Mikrosphäre, einer Tablette in einer Tablette, einer Inlay-Tablette, eines Dragees, eines Beutels oder eines oral verabreichbaren Films vorliegt.

3. Feste orale pharmazeutische Formulierung nach Anspruch 2, wobei die Zusammensetzung in der filmbeschichteten Tablettendosierungsform vorliegt.

4. Feste orale pharmazeutische Formulierung nach Anspruch 2, wobei die Zusammensetzung in der Kapseldosierungsform vorliegt.

5. Feste orale pharmazeutische Formulierung nach einem der vorgenannten Ansprüche, wobei die Zusammensetzung ferner zumindest einen pharmazeutisch akzeptablen Arzneimittelträgerstoff aufweist, der aus Freisetzungsraten-Steuerstoffen, Verdünnungsmitteln, Bindemitteln, Schmiermitteln, Gleitmitteln oder Mischungen derselben ausgewählt ist.

6. Feste orale pharmazeutische Formulierung nach Anspruch 5, wobei die Zusammensetzung zumindest einen Freisetzungsraten-Steuerstoff aufweist, der aus der Gruppe ausgewählt ist, die Hydroxyalkylzellulose, Polyethylenoxid, Alkylzellulose, Carboxymethylzellulose, Polyethylenglykol, Polysacchariden, Natriumalginat, Propylenglykolalginat, Xanthangummi, Carrageenen, Acrylpolymeren und -copolymeren, Carbomeren, Polymethacrylaten, Polyvinylazetat/povidon, Glycerylbehenat, Ethylenvinylazetatcopolymer oder Mischungen derselben aufweist.

7. Feste orale pharmazeutische Formulierung nach Anspruch 6, wobei die Menge des Freisetzungsraten-Steuerstoffs aus der Gruppe ausgewählt ist, die Glycerylbehenat, Polyvinylazetat und einer povidonbasierten Matrix und Carbomer aufweist.

8. Feste orale pharmazeutische Formulierung nach Anspruch 6 oder 7, wobei die Zusammensetzung zumindest ein Bindemittel aufweist, das aus der Gruppe ausgewählt ist, die Laktosemonohydrat, Copovidon, Povidon, Carnaubawachs, Pullulan, Polymethacrylat, Glycerylbehenat, Hydroxypropylzellulose (HPC), Carboxymethylzellulose (CMC), Methylzellulose (MC), Hydroxyethylzellulose, Natriumcarboxymethylzellulose (Na-CMC), Carboxymethylzellulosecalcium, Ethylzellulose, Polymetacrylate, Polyethylenoxid, Polyvinylalkohol, Polycarbophil, Polyvinylazetat und seine Copolymere, Xanthangummi, Guargummi, Alginat, Carrageen, Kollagen, Agar, Pektin, Hyaluronsäure, Carbomer, Zelluloseazetatphtalat, Poloxamer, Polyethylenglykol (PEG), Zucker, Glykosesirupe, natürlichen Gummi, Tragakanthgummi, Polyacrylamid, Aluminiumhydroxid, Bentonit, Laponit, Setostearylalkohol, Polyoxyethylenalkylether, Akazienschleim, Polydextrose oder Mischungen daraus aufweist.

9. Feste orale pharmazeutische Formulierung nach Anspruch 8, wobei das Verhältnis des Bindemittels zu dem Freisetzungsraten-Steuerstoff in dem Bereich von 1:0,2 bis 1:60 und vorzugsweise 1:0,5 bis 1:10 liegt.

10. Feste orale pharmazeutische Zusammensetzung nach Anspruch 3, wobei die Zusammensetzung aufweist:
5-70 Gewichtsprozent Desvenlafaxinsuccinat-Monohydrat,
5-30 Gewichtsprozent Glycerylbehenat,
5-30 Gewichtsprozent eines Matrixstoffs, der Polyvinylazetat in einer Menge von 70-90 % und Povidon in einer Menge von 10-30 % aufweist,
1-50 Gewichtsprozent Laktosemonohydrat,
0,1-10 Gewichtsprozent Natriumstearylfumarat,
0,1-10 Gewichtsprozent kolloidales Siliziumdioxid.

11. Feste orale pharmazeutische Zusammensetzung nach Anspruch 3, wobei die Zusammensetzung aufweist:
5-70 Gewichtsprozent Desvenlafaxinsuccinat-Monohydrat,
10-60 Gewichtsprozent Carbomer,
1-50 Gewichtsprozent Laktosemonohydrat,
0,1-10 Gewichtsprozent Natriumstearylfumarat,
0,1-10 Gewichtsprozent kolloidales Siliziumdioxid.

12. Feste orale pharmazeutische Formulierung nach Anspruch 10 oder 11, wobei die Zusammensetzung ferner eine Beschichtung aufweist, die Polyvinylalkohol, Polyethylenglykol, Titandioxid, Talk und Eisenoxid aufweist.

13. Feste orale pharmazeutische Zusammensetzung nach Anspruch 4, wobei die Zusammensetzung aufweist:
5-70 Gewichtsprozent Desvenlafaxinsuccinat-Monohydrat,
5-30 Gewichtsprozent Glycerylbehenat,
5-30 Gewichtsprozent eines Matrixstoffs, der Polyvinylazetat in einer Menge von
70-90 % und Povidon in einer Menge von 10-30 % aufweist,
1-50 Gewichtsprozent Laktosemonohydrat,
0,1-10 Gewichtsprozent Natriumstearylfumarat,
0,1-10 Gewichtsprozent kolloidales Siliziumdioxid.

14. Feste orale pharmazeutische Zusammensetzung nach Anspruch 4, wobei die Zusammensetzung aufweist:
5-70 Gewichtsprozent Desvenlafaxinsuccinat-Monohydrat,
10-60 Gewichtsprozent Carbomer,
1-50 Gewichtsprozent Laktosemonohydrat,
0,1-10 Gewichtsprozent Natriumstearylfumarat,
0,1-10 Gewichtsprozent kolloidales Siliziumdioxid.

## Revendications

1. - Formulation pharmaceutique orale solide comprenant du succinate de desvenlafaxine monohydraté.

2. - Formulation pharmaceutique orale solide selon la revendication 1, dans laquelle la composition se présente sous forme de comprimé enrobé, de comprimé tricouches, de comprimé bicouches, de comprimé multicouches, de comprimé à désagrégation orale, de mini comprimé, de pastille, de pastille de sucre, de comprimé buccal, de comprimé sublingual, de comprimé effervescent, de comprimé à libération immédiate, de comprimé à libération modifiée, de comprimé pelliculé, de comprimé à désagrégation gastrique, de pilule, de gélule, de granulé oral, de poudre, de système de billes enrobées, de microsphère, de comprimé-dans-comprimé, de comprimé incrusté, de dragée, de sachet ou de film administrable par voie orale.

3. - Formulation pharmaceutique orale solide selon la revendication 2, dans laquelle la composition est sous la forme posologique de comprimé pelliculé.

4. - Formulation pharmaceutique orale solide selon la revendication 2, dans laquelle la composition est sous la forme posologique de gélule.

5. - Formulation pharmaceutique orale solide selon l'une quelconque des revendications précédentes, dans laquelle la composition comprend en outre au moins un excipient pharmaceutiquement acceptable choisi parmi les agents contrôlant la vitesse de libération, les diluants, les liants, les lubrifiants, les agents de glissement ou les mélanges de ceux-ci.

6. - Formulation pharmaceutique orale solide selon la revendication 5, dans laquelle la composition comprend au moins un agent contrôlant la vitesse de libération qui est choisi dans le groupe comprenant l'hydroxy alkyl cellulose, le poly(oxyde d'éthylène), l'alkyl cellulose, la carboxyméthyl cellulose, le polyéthylène glycol, les polysaccharides, l'alginate de sodium, l'alginate de propylène glycol, la gomme de xanthane, les carraghénines, les polymères et copolymères acryliques, les carbomères, les polyméthacrylates, le poly(acétate de vinyle)/povidone, le béhénate de glycéryle, les copolymères éthylène acétate de vinyle ou les mélanges de ceux-ci.

7. - Formulation pharmaceutique orale solide selon la revendication 6, dans laquelle l'agent contrôlant la vitesse de libération est choisi dans le groupe comprenant le béhénate de glycéryle, une matrice à base de poly(acétate de vinyle) et de povidone et les carbomères.

8. - Formulation pharmaceutique orale solide selon l'une des revendications 6 ou 7, dans laquelle la composition comprend au moins un liant qui est choisi dans le groupe comprenant le lactose monohydraté, la copovidone, la povidone, la cire de carnauba, le pullulane, le polyméthacrylate, le béhénate de glycéryle, l'hydroxypropyl cellulose (HPC), la carboxyméthyl cellulose (CMC), la méthyl cellulose (MC), l'hydroxyéthyl cellulose, la carboxyméthyl cellulose sodique (Na CMC), la carboxyméthyl cellulose calcique, l'éthyl cellulose, les polyméthacrylates, le poly(oxyde d'éthylène), l'alcool polyvinylique, le polycarbophile, le poly(acétate de vinyle) et ses copolymères, la gomme de xanthane, la gomme de guar, l'alginate, le carraghénane, le collagène, la gélose, la pectine, l'acide hyaluronique, les carbomères, l'acétate phtalate de cellulose, les poloxamères, le polyéthylène glycol (PEG), les sucres, les sirops de glucose, les gommes naturelles, la gomme adragante, le polyacrylamide, l'hydroxyde d'aluminium, le bentonite, la laponite, l'alcool cétostéarylique, les alkyl éthers polyoxyéthylénés, le mucilage d'acacia, le polydextrose ou les mélanges de ceux-ci.

9. - Formulation pharmaceutique orale solide selon la revendication 8, dans laquelle le rapport du liant à l'agent contrôlant la vitesse de libération est dans la plage de 1:0,2 à 1:60 et, de préférence, de 1:0,5 à 1:10.

10. - Composition pharmaceutique orale solide selon la revendication 3, dans laquelle la composition comprend :
- 5 à 70 % en poids de succinate de desvenlafaxine monohydraté ;
- 5 à 30 % en poids de béhénate de glycéryle ;
- 5 à 30 % en poids d'un agent de matrice qui comprend du poly (acétate de vinyle) dans une quantité de 70 à 90 % et de la povidone dans une quantité de 10 à 30 % ;
- 1 à 50 % en poids de lactose monohydraté ;
- 0,1 à 10 % en poids de fumarate de stéaryle et de sodium ;
- 0,1 à 10% en poids de dioxyde de silicium colloïdal.

11. - Composition pharmaceutique orale solide selon la revendication 3, dans laquelle la composition comprend :
- 5 à 70 % en poids de succinate de desvenlafaxine monohydraté ;
- 10 à 60% en poids de carbomère ;
- 1 à 50 % en poids de lactose monohydraté ;
- 0,1 à 10% en poids de fumarate de stéaryle et de sodium ;
- 0,1 à 10 % en poids de dioxyde de silicium colloïdal.

12. - Formulation pharmaceutique orale solide selon l'une des revendications 10 ou 11, la composition comprenant en outre un enrobage comprenant de l'alcool polyvinylique, du polyéthylèneglycol, du dioxyde de titane, du talc, de l'oxyde de fer.

13. - Composition pharmaceutique orale solide selon la revendication 4, dans laquelle la composition comprend :
- 5 à 70 % en poids de succinate de desvenlafaxine monohydraté ;
- 5 à 30 % en poids de béhénate de glycéryle ;
- 5 à 30 % en poids d'un agent de matrice qui comprend du poly (acétate de vinyle) dans une quantité de 70 à 90 % et de la povidone dans une quantité de 10 à 30 % ;
- 1 à 50 % en poids de lactose monohydraté ;
- 0,1 à 10 % en poids de fumarate de stéaryle et de sodium ;
- 0,1 à 10 % en poids de dioxyde de silicium colloïdal.

14. - Composition pharmaceutique orale solide selon la revendication 4, dans laquelle la composition comprend :
- 5 à 70 % en poids de succinate de desvenlafaxine monohydraté ;
- 10 à 60 % en poids de carbomère ;
- 1 à 50 % en poids de lactose monohydraté ;
- 0,1 à 10% en poids de fumarate de stéaryle et de sodium ;
- 0,1 à 10% en poids de dioxyde de silicium colloïdal.
